# EUROPÄISCHE PATENTANMELDUNG

(11) **EP 1 707 639 A1**
(43) Veröffentlichungstag der Anmeldung: **04.10.2006**
(21) Anmeldenummer: 05018157.7
(22) Anmeldetag: 23.08.2005
(51) Int. Cl.: C12Q 1/68

(54) **Verfahren für den Nachweis von Genmutationen durch sequenzspezifisches Schneiden, Markieren und Grössenmessung der markierten Fragmente**

(30) Priorität: 31.03.2005 DE 102005015037
(71) Anmelder: Nagel, Mato, 02943 Weisswasser (DE)
(72) Erfinder: Nagel, Mato, 02943 Weisswasser (DE)

(57) **Zusammenfassung**

Automatisierbares Verfahren zum Nachweis ausgedehnter Genmutationen (Rearrangements, Insertionen, Deletionen, Inversionen und Duplikationen) basierend auf der Kombination und gezielten Variation der sequenzspezifischen Prozesse von Erzeugung unterschiedlich großer Fragmente durch sequenzspezifische Endonukleasen und einer sequenzspezifischen Größenmessung dieser Fragmente.

## Beschreibung

### 1. Einleitung

Die Untersuchung der genetischen Information gewinnt in der medizinischen Diagnostik einen immer größeren Stellenwert. Während die Untersuchung von kleineren Mutationen mittels Sequenzierung inzwischen einen hohen Automatisierungsgrad erreicht hat, sind nach wie vor die großen Mutationen schlecht zu erfassen. Der Sequenzierung entgehen sie meist, weil sie über die kleinen sequenzierbaren Bereiche hinausgehen. Eine Methode nach welcher solche großen Mutationen entdeckt werden können ist der Southern-Blot. Diese Methode ist allerdings sehr aufwendig, was zum einen die erforderlichen nur manuell zu erledigende

Arbeitsschritte und die aufwendigen zum Teil radioaktiven Reagenzien betrifft. Dies führt dazu, dass auf der einen Seite eine Fülle neuer Kleinmutationen entdeckt und in der wissenschaftlichen Literatur beschrieben werden, aber auf der anderen Seite die Zahl der bekannten Großmutationen nahezu stagniert. Für die Patienten bedeutet dies, dass eine große Zahl ihrer Mutation derzeit nicht entdeckt werden können. Mit dem hier beschriebenen Verfahren wird eine Möglichkeit eröffnet diese Mutationen in ähnlicher Weise wie beim

Souther-Blot zu erfassen. Da der Nachweis allerdings in der flüssigen Phase erfolgt, sind nicht wie beim Southern-Blot die aufwendigen und störanfälligen Prozeduren der Fixierung des

Untersuchungsmaterials erforderlich und eine Automatisierung möglich.

### 2. Methodik

### 2.1. Allgemeine Bemerkungen zum Verfahren

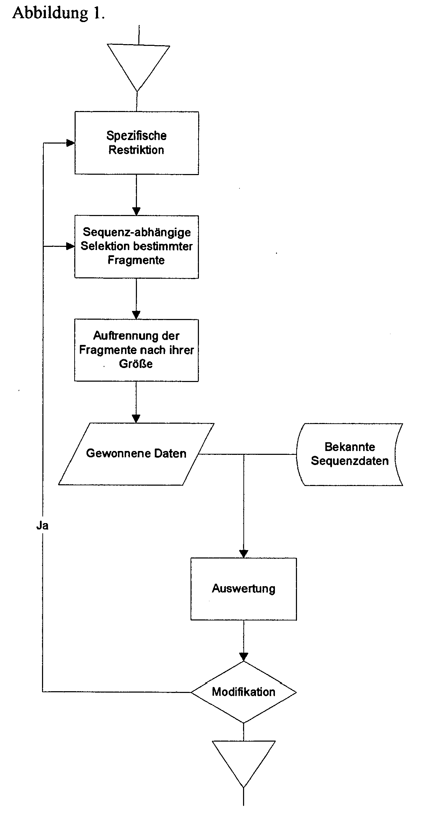

Das Verfahrens basiert auf die sequenzielle Einwirkung zweier sequenzspezifischer Prozesse
**[Abbildung 1 Die Zeichnung stellt das Ablaufschema des Verfahrens dar.]**
1. Die Restriktion durch Endonukleasen, die native DNA an ganz speziellen bekannten und benannten Restriktionsstellen zu spalten vermögen.
2. Die sequenzspezifische Markierung durch kurze sequenzspezifisch reagierende Moleküle (DAN, RNA, PNA), die sich nur an komplementären DNA-Strängen anzulagern vermögen.

Voraussetzung für die gezielte Anwendung dieser Prozesse ist die Wissensbasis der Genomsequenzen. Durch die wiederholte und modifizierte Anwendung dieser Prozesse lassen sich die gewünschten Informationen immer weiter eingrenzen bis es zur diagnostischen Beurteilung eines speziellen Falls ausreicht.
Der grundsätzliche Ablauf umfasst zwei Schritte:
1. Der erste Schritt beinhaltet die Aufspaltung nativer DNA durch sequenzspezifische Restriktionsenzyme. Hierdurch entsteht eine große Anzahl von DNA Fragmenten unterschiedlicher Größe und unterschiedlicher Basesequenz. Normalerweise ist davon auszugehen, dass Bruchstücke mit gleicher Basensequenz auch die exakt gleiche
   Größe aufweisen.
2. Im zweiten Schritt erfolgt nun die Größenmessung von Fragmenten gleicher Basensequenz. Sollte nicht die erwartete Größe bestimmt werden, so ist von einer Mutation auszugehen.

Dieses Verfahren erlaubt nicht nur die grundsätzliche Aussage, ob eine Mutation vorhanden ist oder nicht. Durch modifizierte Wiederholung der Untersuchung lässt sich die zugrundeliegende Mutation immer genauer charakterisieren. Man wird diesen Iterationsprozess allerdings nur so weit treiben, bis die klinische oder wissenschaftliche Fragestellung hinreichend beantwortet werden kann.

Im wesentlichen lassen sich zwei Modifikationen dieses Verfahrens unterscheiden:
1. Die Markierung der Fragmente erfolgt in der flüssigen Phase und die Größenbestimmung der markierten Fragmente durch eine größenabhängige Auftrennung, wie der Elektrophorese.
2. Die komplementären Molekule (DNA, RNA, PNA), welche sich mit einem spezifischen Fragment verbinden, sind an einer festen Phase fixiert und die Anlagerung der Fragmente wird als Massezunahme an diesem Punkt gemessen. Dabei ist die Massenzunahme proportional der Größe des sich anlagernden Fragmentes. Diese Massenbestimmung könnte anhand von surface acoustic waves erfolgen.

Eine weitere Unterteilung nach Art der Markierung und dem Verfahren der Detektion ist möglich. Eine Übersicht der methodischen Variationen gibt die folgende Gliederung.
1. Detektionsverfahren für große Rearrangements
   a. Komplementärsequenz an fester Phase gebunden in unmittelbarer Nähe der Detektoren
      i. mechanisch/akustisches Detektionsverfahren
      ii. elektrisches Detektionsverfahren
   b. Komplementärsequenz in flüssiger Phase mit einem detektierbaren Signalgeber
      i. Flourezierender Signalgeber
      ii. magnetischer Signalgeber

### 2.1.2. Größenbestimmung mittels Kapillarelektrophorese

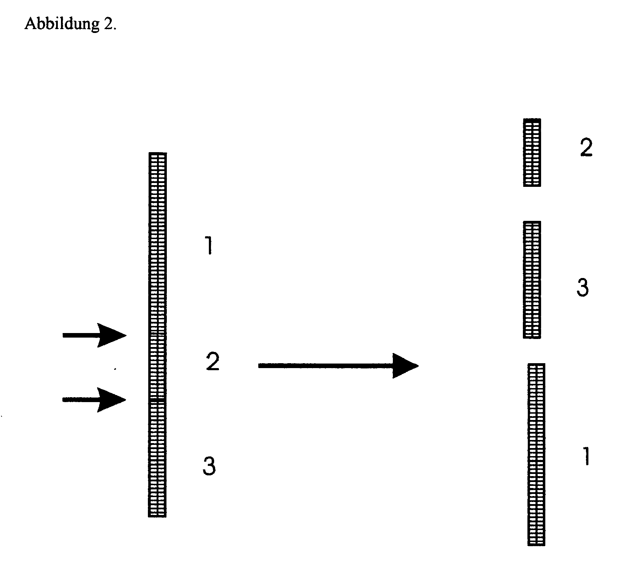

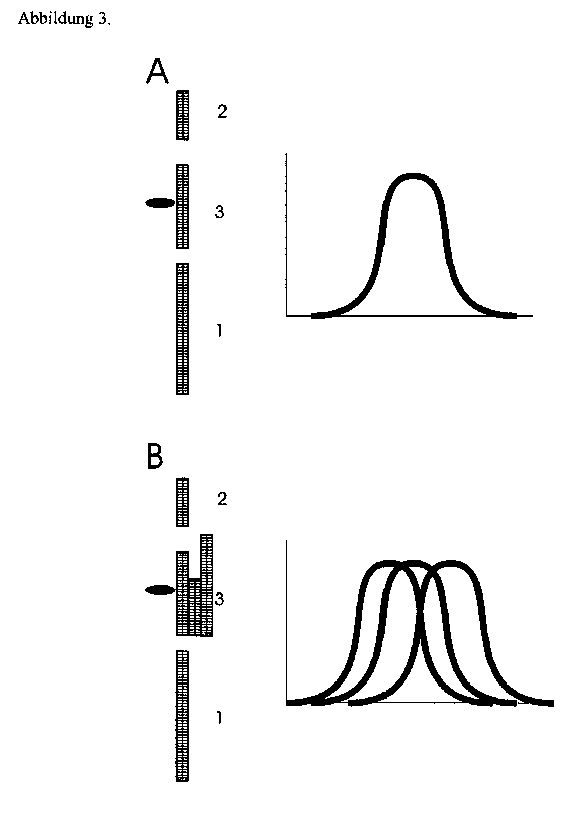

Der Ablauf besteht im wesentlichen aus folgenden Schritten:
1. Im ersten Schritt wir die native zu untersuchende DNA durch ein spezifisches Restriktionsenzym in kleinere Bruchstücke unterschiedlicher Größe gespalten
   **[Abbildung 2 Die native doppelsträngige DNA wird durch ein sequenzspezifisches Restriktionsenzym an den mit roten Pfeilen markierten Stellen in Bruchstücke unterschiedlicher Größe aufgespaltet. Diese wandern am Sensor der Kapillarelektrophorese sortiert nach ihrer Größe (die kleineren zuerst) vorbei. Hier in der Abbildung entspricht dies der Reihenfolge 2-3-1.]**
2. Nun wird eines der Bruchstücke mit einem der weiter unten zu beschreibenden Verfahren markiert, dass es ein mit einem beliebigen Sensor erfassbares Signal erzeugen kann.
3. Diese Bruchstücke werden nun entsprechen ihrer Größe aufgetrennt. Es existieren hierzu bereits eine Fülle von Verfahren in der Molekularbiologie. Ein probates und bereits gut automatisiertes Verfahren ist die Kapillarelektrophorese, anhand welcher das hier vorgestellte Verfahren der Detektion großer Mutationen im weiteren erläutert werden soll. Die Bruchstücke wandern in der Kapillarelektrophorese in Abhängigkeit von ihrer Größe an einem Sensor vorbei. Die kleinen Bruchstücke erreichen dabei den Sensor schneller als die großen. Da diese Bruchstücke allerdings keine Markierung besitzen erzeugen sie am Sensor auch kein Signal. Nur das markierte Bruchstück erzeugt eine Signalspitze an einer bestimmten Stelle der Zeitachse (Abbildung 3 A).
4. Tritt nun eine große Mutation in dem markierten Abschnitt auf, so verschiebt sich die Position des Signals entweder nach vom (Deletion) oder nach hinten (Insertion/Duplikation) (Abbildung 3 B).
   **[Abbildung 3 Nach Markierung ergibt das Bruchstück 3 am Sensor ein Signal (A) Dieses Signal verschiebt sich nach vorn (grün) oder nach hinten (blau) je nachdem, ob das Bruchstück 3 durch Deletion verkürzt oder durch Insertion oder Duplikation verlängert ist (B).]**
5. Verschiedene Restriktionsenzyme erzeugen unterschiedliche Bruchstücken, die sich sehr genau charakterisieren lassen. So kann man durch Kombination verschiedener Markierungen mit unterschiedlichen Restriktionsenzymen ein ziemlich exaktes Bild über die Art und Ausdehnung der Mutation gewinnen.

### 2.1.1. Größenbestimmung mittels Surface acoustic waves

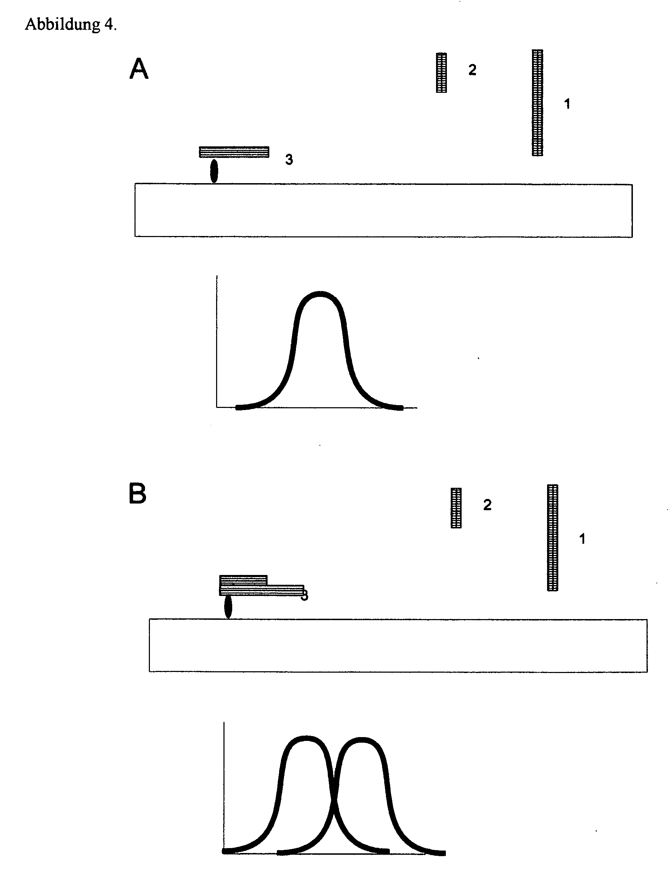

Der Ablauf besteht im wesentlichen aus folgenden Schritten:
1. Im ersten Schritt wir die native zu untersuchende DNA durch ein spezifisches Restriktionsenzym in kleinere Bruchstücke unterschiedlicher Größe gespalten (Abbildung 2).
2. An eine Oberfläche, die mit surface acoustic waves untersucht werden kann (Quarz), werden unterschiedliche sequenzspezifische Komponenten (DNA-, RNA-Oligonucleotide, PNA) gebunden, so dass genau bekannt ist, am welcher Stelle welches Fragment der gespaltenen nativen DNA anlagert.
3. Die Lösung mit den Verschiedenen Fragmenten nativer DNA wird darüber gegeben und so gewaschen, dass nur die speziefisch angelagerten Fragmente haften bleiben.
4. Anschließend wird mittels akustischer oder elektrischer Meßmethode die Masse der Angelagerten DNA bestimmt und damit die Größe des Fragmentes berechnet (Abbildung 4 A).
6. Tritt nun eine große Mutation in dem angelagerten Fragment nativer DNA auf, so verschiebt sich die Position des Massesignals entweder in Richting kleinerer (Deletion) oder größerer Masse (Insertion/Duplikation) (Abbildung 4 B).
   **[Abbildung 4 Eine feste Phase (Quarz) ist an verschiedenen Stellen mit unterschiedlichen Komponenten bestückt, die sich sequenzspezifisch mit ganz bestimmten Fragmenten der gespaltenen nativen DNA verbinden. Wird die angelagerte Masse an solch einem Punkt vermessen ergibt sich ein definiertes Massesignal (A) Dieses Signal verschiebt sich zur geringeren (grün) oder größeren Masse hin (blau) je nachdem, ob das Bruchstück 3 durch Deletion verkürzt oder durch Insertion oder Duplikation verlängert ist (B).]**
5. Verschiedene Restriktionsenzyme erzeugen unterschiedliche Bruchstücken, die sich, weil wir die Genomdaten kennen, sehr genau berechnen lassen. So kann man aus der Kombination verschiedener sequenzspezifischer Komponenten mit unterschiedlichen Restriktionsenzymen ein ziemlich exaktes Bild über die Art und Ausdehnung der Mutation gewinnen.

### 2.2. Verfahren zur Markierung

Die hier beschriebenen Markierungsverfahren stellen nur eine Auswahl der wichtigsten Methoden dar. Die Darstellung erfolgt nur im Prinzip. Die Arbeitsschritte bei der Präparation sind nicht im einzelnen dargestellt.

### 2.2.1. Markierung durch komplementäre markierte DNA

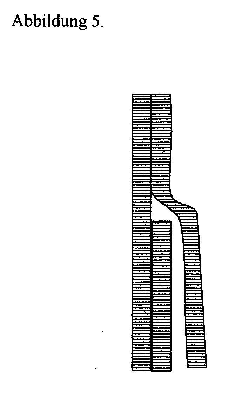

Dies ist das einfachste Verfahren. Es kommen kürzere DNA-Stücke von 20-150bp zur Anwendung. Diese durch eine Oligonucleotidsynthese oder PCR-Reaktion gewonnenen Marker müssen anschließend gut gereinigt werden. Die Markierung erfolgt entweder direkt bei der Synthese oder anschließend mit bereits kommerziell erhältlichen Kits. Zur Markierung der nativen DNA wird eine definierte Menge der hier gewonnenen markierten komplementären DNA hinzu gegeben, denaturiert, anschließend wieder renaturiert und gereinigt. Die markierte komplementäre DNA hat sich dann mit einem spezifischen Bruchstück der nativen DNA verbunden und bringt dieses dann am Sensor zur Detektion
**[Abbildung 5) Das einsträngige Stück markierter DNA (rot) lagert sich zwischen die doppelsträngige native DNA (schwarz) und markiert damit dieses Bruchstück.]**

### 2.2.2. Markierung durch komplementäre PNA

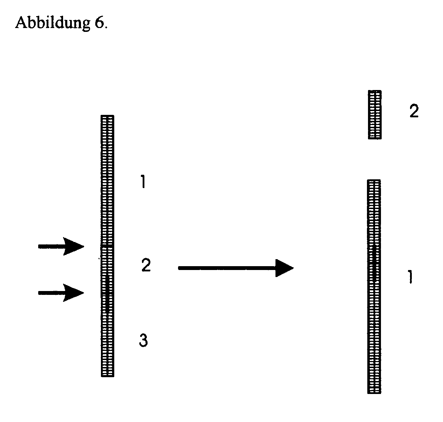

Die Markierung mit komplementärer PNA (peptide nucleic acid) ist nur eine dahingehende Modifikation des vorhergehenden Verfahrens, dass statt markierter DNA PNA verwendet wird. PNAs sind stabiler und binden fester als DNA und sind damit in der Lage native DNA aus ihrer komplementären Bindung zu verdrängen. Zusammen mit ihrer Eigenschaft der Resistenz gegenüber Restriktionsenzymen ergibt sich eine interessante Modifikation des Verfahrens dahingehend, dass eine PNA genau so erstellt wird, dass eine spezifische Restriktionsstelle blockiert wird. Wenn Sie dann vor dem ersten Arbeitsschritt zur nativen DNA hinzu gegeben wird, entsteht ein ganz spezifisches markiertes DNA-Fragment, welches auch in leicht denaturierenden Kapillarelektrophoresegelen nachgewiesen werden kann
**[Abbildung 6. Durch die Anlagerung des PNA Stückes zwischen die beiden nativen DNA Stränge an der Restriktionsstelle zwischen den sonst üblichen Fragmenten 2 und 3 wird die Restriktion an dieser Stelle verhindert und es entsteht ein großes markiertes Fragment.]**
Dies kann eine besondere Bedeutung bei schwer abzugrenzenden repetitiven Elementen bekommen.

### 2.2.3. Markierung durch PCR mit markierten Primern

Die Markierung von DNA Bruchstücken mittels PCR kann sowohl bidirektional besser jedoch unidiketional erfolgen. Dabei erzeugt die Elongation des Primers am Template der nativen DNA ein langes und damit nicht nur spezifisch sondern auch sehr fest haftende komplementäre markierte DNA.

### 2.2.3. Markierung durch Signalgeber an magnetischen (Spin-)Sensoren

Die magnetische Markierung hat den Vorteil, dass die Sensitivität und die Variabilität erheblich erhöht werden können. In Betracht gezogen werden Fullerene, wo eine enorme Menge unterschiedlicher magnetischer Inhalte differenziert werden können, so dass eine gleichzeitige Differenzierung sehr viel verschiedener DNA Fragmente in einem einzigen Untersuchungsablauf möglich ist. Die Floureszenzmarkierung bietet Im Gegensatz hierzu nur eine sehr begrenzte Auswahl an differenzierbaren Floureszenzfarbstoffen.
Eine weitere Modifikation wäre die einfache Anlagerung verschiedener Metallionen an unterschiedliche sequenzspezifische Oligonukliotide. Die mit den gleichen magnetischen Detektoren erfasst werden können. Während dies einerseits gegenüber den Fullerenen eine Vereinfachung darstellt sind jedoch der Flexibilität grenzen gesetzt. Zudem sind die freiliegenden Metallionen nicht chemisch innert, wie die Fullerene.

### 2.3. Verfahren der Restriktion

Der Abschnitt dient der Erläuterung der Flexibilität des zu patentierenden Verfahrens.

### 2.3.1. Verschiedene Restriktionsenzyme

Es sind bereits eine Reihe von Restriktionsenzymen bekannt und kommerziell verfügbar, die doppelsträngige DNA sequenzabhängig spalten können. Die dabei spezifisch erkannte Sequenz ist allerdings so kurz, dass die native DNA an sehr vielen Stellen gespalten wird und somit eine Fülle unterschiedlich großer Fragmente entsteht.
Der Prozess der Restriktion selbst ist einfach durchführbar und durchaus automatisierbar. Durch die Wahl eines speziellen Restriktionsenzyms kann genau der gewünschte DNA Bereich fragmentiert werden.

### 2.3.2. Modifikation der Restriktion

Eine Modifikation der Restriktion ist unter der PNA-Markierung bereits erwähnt. Weitere Möglichkeiten beständen in der gezielten Methylierung. Durch sequenzspezifische Methyltransferasen.

### 2.3.3. Hochspezifische Restriktionsenzyme

Neben den bekannten natürlichen, vornehmlich aus Mikroorganismen gewonnenen Restriktionsenzymen, die inzwischen meist gentechnisch hergestellt werden können. Sind auch hochspezifische Restriktionsenzyme in der Entwicklung. Diese neuen Restriktionsenzyme spalten dann die DNA nicht an vielen sondern nur an wenigen oder gar an einer einzelnen Restriktionsstelle. Durch die Verfügbarkeit solcher Enzyme gewinnt das hier beschriebene Verfahren natürlich zusätzlich gewaltig an Bedeutung.

### 2.3.3. Hochspezifischer Bruch der DNA-Kette durch sequenzspezifische Kraftübertragung mittels magnetischer sequenzspezifischer Komponenten

Spezifische DNA oder PNA lagert sich sequenzspezifisch an die native DNA. Wenn nun diese sequenspezifische Komponente an magnetisierbare Komponenten (Nanotubes, Fullerene, Metallionen) gekoppelt ist, so besteht durch Anlegen eines externen schwingenden Magnetfeldes die Möglichkeit diesen Komplex zum Schwingen zu bringen. Dies bedeutet eine ganz lokalisierte enorme Kraftübertragung auf die native DNA, die letztendlich zu ganz spezifischen Strangbrüchen in der nativen DNA führt. Damit lassen sich zur Vervollkommnung der Gesamtmethode Fragmente erzeugen, die in ihrer Einmaligkeit der Gesamtmethode einen sehr hohen Informationsgehalt beisteuern.

## Patentansprüche

1. Der Patentanspruch umfasst vor allem das Prinzip nach welchem die großen Mutationen in automatisierbaren Verfahren nachgewiesen werden können. Der Patentanspruch umfasst also den automatisierbaren technologischen Ablauf von
a. Spaltung nativer, zu untersuchender DNA
b. Größenbestimmung von Fragmente mit spezifischer DNA-Sequenz, durch verschiedene og. Methoden.
c. Auswertung mit Hinweisen für eine Modifikation der technologischen Schritte a und b.

2. Die Markierung mit komplemantärer PNA vor der Restriktion stellt eine erfinderische neue Kombination der Nutzung von PNA und Restriktionsenzymen dar. Da dies eine besondere diagnostische Erweiterung des Repertoires des hier zu schützenden Verfahrens darstellt, sollte es ebenfalls unter Schutz gestellt werden.

3. Gleichermaßen wird der Schutz beansprucht für die Idee der durch sequenzspezifisch angelagerte magnetisierbare Komponenten ermöglichten eng lokalisierten Kraftübertragung und damit hervorgerufener Brechung der DNA-Kette.

4. Desgleichen wird eine Schutz für Markierungen beansprucht, die durch magnetische Sensoren detektiert werden können (Fullerene, Metallionen). Da diese durch ihre hohe Sensitivität eine große Bedeutung für dieses Verfahren besitzen.

5. Ein Patentanspruch wird im Zusammenhang mit dem genannten Verfahren auch für die spezifische Größenbestimmung von DNA Fragmenten mittels dem Verfahren der Surface Acoustic Waves beantragt.
